# EUROPEAN PATENT SPECIFICATION

(11) **EP 2 146 750 B1**
(45) Date of publication and mention of the grant of the patent: **13.07.2011**
(21) Application number: 08767142.6
(22) Date of filing: 07.05.2008
(51) Int. Cl.: A61K 47/48, A61K 38/27, A61K 38/28, A61K 31/728

(54) **STABILIZED SUSPENSION**
STABILISIERTE SUSPENSION
SUSPENSION STABILISÉE

(30) Priority: 07.05.2007 SE 0701090; 07.05.2007 US 924257 P
(43) Date of publication of application: 27.01.2010
(73) Proprietor: Jederstrom Pharmaceuticals AB, 104 35 Stockholm (SE)
(72) Inventor: JEDERSTRÖM, Gustaf, S-113 40 Stockholm (SE); JEDERSTRÖM, Mikael, S-195 34 Märsta (SE)
(74) Representative: Nikolopoulou, Sofia
(86) International application number: PCT/SE2008/050531
(87) International publication number: WO 2008/136759

(56) References cited:
- WO-A1-97/37680
- WO-A2-01/36656
- JEDERSTRÖM GUSTAF ET AL: "Blood glucose-lowering activity of a hyaluronan-insulin complex after oral administration to rats with diabetes." December 2005 (2005-12), DIABETES TECHNOLOGY & THERAPEUTICS DEC 2005 LNKD- PUBMED:16386101, VOL. 7, NR. 6, PAGE(S) 948 - 957 , XP002577589 ISSN: 1520-9156 * page 954 - page 956 *
- JEDERSTRÖM G. ET AL.: 'Formulating Insulin for Oral Administration: preparation of Hyaluronan-Insulin Complex' PHARMACEUTICAL RESEARCH vol. 21, no. 11, November 2004, pages 2040 - 2047, XP003023874
- YOSHIOKA S. ET AL.: 'Correlations between Molecular Mobility and Chemical Stability During Storage of Amorphous Pharmaceuticals' JOURNAL OF PHARMACEUTICAL SCIENCES vol. 96, no. 5, May 2007, pages 960 - 981, XP003023875
- WANG W. ET AL.: 'Lyophilization and development of solid protein pharmaceuticals' INTERNATIONAL JOURNAL OF PHRMACEUTICS vol. 203, 2000, pages 1 - 60, XP002428586
- PIKAL M.J. ET AL.: 'The stability of Insulin in Cristalline and Amorphous Solids: Observation of Greater Stability for the Amorphous Form' PHARMACEUTICAL RESEARCH vol. 14, 1997, pages 1379 - 1387, XP003023877
- ZHANG Y. ET AL.: 'Trehalose and hyaluronic acid coordinately stabilized freeze-dried pancreatic kininogenase' EUROPEAN JOURNAL OF PHARMACEUTICS AND BIOPHARMACEUTICALS vol. 65, 2007, pages 18 - 25, XP005788721

## Description

Stabilized, colloidal free radical reduced aqueous suspension containing a complex of hyaluronic acid and a protein drug, a pharmaceutical composition, and a method of producing the same.

The present invention relates to a stabilized, colloidal free radical reduced aqueous suspension containing hyaluronic acid and a protein, to a pharmaceutical composition comprising said suspension, and to a method of producing the same. More precisely, the invention relates to a stabilized, colloidal aqueous suspension containing a complex of amorphous hyaluronic acid (HA) and an amorphous protein drug, such as insulin, glucagon-like peptide 1, human growth hormone, erythropoietin or super oxide dismutase. The pharmaceutical composition of the invention is intended for oral as well as parenteral administration. The method of the invention comprises production of amorphous hyaluronic acid and amorphous protein.

### Background of the invention

The pharmaceutical industry is continuously interested in finding transport and storage stabile pharmaceutical preparations containing active ingredients, such as protein or peptide drugs. Further, one of the main challenges in research associated with the medical use of proteins and peptides is to find a formulation suitable for oral administration.

Orally administered proteins are normally rapidly destroyed in the alimentary canal by proteases, peptidases, and/or gastric acid. Moreover, the gut epithelium is an effective barrier, which prevents, both physically and as a result of the local digestive enzymes, the uptake of proteins into the systemic circulation. As a consequence, very few functional pharmaceuticals containing proteins intended for oral administration have yet been developed.

Consequently, protein and peptide drugs are presently administered preferably parenterally, e.g. by subcutaneous injection or by pulmonary administration, which is often accompanied by inconvenience for the patients. Further, peptides and proteins for parenteral administration are still subject to short half-life and loss of activity problems. Thus, in addition to protecting pharmaceuticals containing proteins or peptides from digestion, their absorption in the body, and consequent transport to the target organs, must be ensured. The proteins or peptides must also be delivered to the target organs and the receptors in an active form. Efficient oral dosage forms would widen the therapeutic window by influencing the distribution of the drug by use of the enterohepatic circulation (EHC). Drugs entering the intestinal tract will normally be absorbed into the portal circulation, from where the drugs can be removed from systemic circulation by hepatic uptake for an improved, even distribution in man.

Hyaluronic acid (also known as hyaluronan) is an endogenous, naturally occurring glycosaminoglycan, in the form of sodium hyaluronate, with a documented safety record for use *in vivo* as a carrier for protein drugs. It consists of a linear polymer of repeating units of glucuronic acid and N-acetyl-glucosamine. The molecular weight can vary over a wide range depending on the source. Hyaluronic acid is present in several tissues of mammals, and in some specific tissues, such as rooster combs, in concentrations high enough for commercial scale extraction. Such tissues contain hyaluronic acid of a wide range of molecular weights. Hyaluronic acid may also be obtained from bacteria, e.g. recombinant Hyaluronan, and can be purified during a complex series of extraction, purification and sterilization steps. This results in a final product having a high molecular weight within a considerably narrower molecular weight range.

In WO 9005522, hyaluronic acid is mentioned as a slow release carrier in a subcutaneously injected to obtain a slow release depot effect, together with a binding protein for e.g. growth hormone (GH) or Insulin-like growth factor (IGF). The active substances are linked to the hyaluronic acid by covalent bonds. Hyaluronic acid has also been used to improve the adhesion of biomolecules to biologic membranes and thus improve the internalization of biomolecules. However, the main medical use of hyaluronic acid is presently the administration of purified hyaluronic acid (of animal origin or synthesized by use of recombinant cells) directly at sites, where either a mechanical functional barrier is required (viscosurgery, viscoprotection, viscoseparation, e.g. in ophthalmic surgery) or where a mechanically dysfunctional tissue can be supplemented (viscosupplementation, viscoaugmentation, e.g. in cosmetic surgery).

Prior to the present invention, complexes of hyaluronan and insulin were described by Jederström et al. in the paper "Formulating Insulin for Oral Administration: Preparation of Hyaluronan-Insulin Complex, Pharmaceutical Research, Vol. 21, No. 11, November 2004. Moreover,the present inventor has in EP 1 231 926 described complexes between a pharmacologically active biomolecule and hyaluronic acid. Such complexes were shown to be effective in penetrating biological membranes, thus avoiding the need for parenteral administration. The complexes were formed by solubilization of hyaluronic acid in a solution containing an acid and an electrolyte solution, whereupon the biolmolecules were dissolved in the dissolved hyaluronic acid and an acid solution, before dialysis of the latter solution to about neutral pH.

Although the above mentioned complexes have shown good medical capabilities with an acceptable storage life at low temperatures of 2 - 8 °C, there is still a need for a stabilized, colloidal aqueous suspension containing hyaluronic acid and a protein drug with a longer storage life, particularly at higher temperatures, especially for use in an orally deliverable pharmaceutical composition.

### Description of the invention

As opposed to the complex of the EP 1 231 926 wherein the hyaluronic acid as well as the biomolecules used as starting materials were in crystalline form, the present invention provides a stabilized, colloidal aqueous suspension containing a complex of amorphous Hyaluronic acid (HA) and an amorphous protein drug, wherein the water content is minimized by an excess amount of HA. Particular features of the present invention are that both the HA and protein drug are in amorphous form as the complex is formed and the minimized water content stabilizes the colloidal suspension so that the product gets an extended storage life even at elevated ambient temperatures. Further, the amorphous forms of hyaluronan and the protein drug in the complex, according to the invention, contain high entropy that will improve absorption *in vivo.*

Thus, a first aspect of the invention is directed to a stabilized, colloidal free radical reduced aqueous suspension containing a complex of amorphous Hyaluronic acid (HA) with a mean molecular weight (mean MW) in the range of 80 to 400 kDa and an amorphous protein drug, and an excess amount of HA to minimize the water content. It should be understood that also the excess amount of HA to minimize the water content could have a mean molecular weight in the range of 80 to 400 kDa.

The term stabilized comprises a stable complex in suspension or a free flowing powder thereof. Moreover, the term stabilized also comprises a stabilized pharmaceutical preparation with considerable storage stability.

The term "protein" comprises in the present invention both monomeric and multimeric polypeptides, such as hormones and enzymes.

By "Hyaluronic acid" or "Hyaluronan" is meant hyaluronic acid both in protonated and non-protonated form, irrespective of origin. The invention thus encompasses the use of hyaluronic acid of both animal and bacterial origin, for example hyaluronic acid produced by genetically modified organisms. In general, the invention encompasses the use of all hyaluronic acid derivatives, which do not compromise the physiological properties associated with hyaluronic acid.

The values of the mean MW of HA in this invention indicate that the majority of the molecular weights of HA are between the indicated lowest and highest values, whereas it should be understood that some of the HA may be of lower or higher MW.

By the aqueous suspension being free radical reduced should be understood herein that the suspension is during part of or during the entire process purged with an inert gas, such as nitrogen. Moreover, glassware is also advantageously treated for removal of free radicals, using processes known in the art. The term "inert conditions" is used herein as a description of a process carried out in an inert gas atmosphere. The reduction of free radicals significantly enhances the shelf life of the resulting product.

In an embodiment of the invention the HA of the excess amount of HA has a mean MW in the range of 6 - 15 kDa.

In another embodiment of the invention, the suspension additionally comprises HA with a mean MW in the range of 1 - 8 kDa and is in lyophilized free-flowing powder form.

In a further embodiment of the stabilized, colloidal aqueous suspension according to the invention the protein drug is selected from the group consisting of insulin, glucagon-like peptide 1(GLP1), human growth hormone (hGH), erythropoietin (EPO) and super oxide dismutase (SOD), but it should be understood that the invention is not limited to these examples and that any protein drug may be used in amorphous form for the purpose of the invention.

Another aspect of the invention is directed to a pharmaceutical composition comprising a stabilized, colloidal aqueous suspension of the invention and a pharmaceutically acceptable carrier.

In an embodiment of this aspect of the invention, the pharmaceutically acceptable carrier is an orally dispensable dosing device for oral administration of the colloidal aqueous suspension. Examples of these include an obelate or an orally deliverable capsule, formulated to contain a free-flowing powder of amorphous complexed protein, such as insulin, and amorphous hyaluronan of different molecular weights, especially the latter one that is designed for enteric delivery of the composition, but any suitable form for oral administration is possible, such as a tablet, or another oral delivery form, including vehicles commonly used for oral preparations.

In another embodiment of the invention the pharmaceutically acceptable carrier is an orally dispensable dosing device for oral administration of the colloidal aqueous suspension. Examples of these include an orally deliverable capsule, especially one that is designed for enteric delivery of the composition, but any suitable form for oral administration is possible, such as a suspension, or other oral delivery form, including vehicles commonly used for oral preparations.

In yet another embodiment of this aspect of the invention the pharmaceutically acceptable carrier is a dosing device for parenteral administration of the colloidal aqueous suspension, such as a vial or syringe.

The pharmaceutical composition of the invention may thus be in different administration forms and may contain additives and excipients approved for delivery to mammals, especially humans, usually contained in pharmaceutical composition, such as those found in the European or US pharmacopoeia.

Yet another aspect of the invention is directed to a method of producing a stabilized, colloidal free radical reduced aqueous suspension of a complex of Hyaluronic acid (HA) and a protein drug, comprising carrying out the following steps:
(a) lyophilizing an aqueous solution of HA with a mean molecular weight (mean MW) in the range of 80 and 400 kDa and maintaining the lyophilization during a sufficient time period, to obtain the HA in amorphous form,
(b) lyophilizing an aqueous solution of the protein drug and maintaining the lyophilization during a sufficient time period, to obtain the protein drug in amorphous form,
(c) admixing the lyophilized, amorphous HA with a mixture of electrolytes and water, whereby electrical double layers are obtained on the HA of the resulting homogenous mixture,
(d) adding the lyophilized, amorphous protein drug at a low pH, such as a pH of 1 - 2; during a time period that is sufficiently short not to denature the protein, whereby electrical double layers are obtained on the amorphous protein drug
(e) reducing the content of water and ions to a minimum, whereby hydrophobic surfaces are drawn to each other to form a complex of the amorphous HA and the amorphous protein drug; and whereby the pH of the complex increases, and
(f) adding an excess amount of HA with a mean MW in the range of 6 - 15 kDa in the minimum of water to produce the stabilized colloidal suspension containing the complex of the amorphous HA and the amorphous protein drug.
It should be realized that protein aggregation is prohibited by an excess of HA in the stabilized, colloidal aqueous suspension of a complex of HA and a protein drug according to the invention.

With regard to the lyophilizations, the lyophilization in step (a) may be carried out at a temperature of - 40°C or lower and/or the lyophilization in step (b) may be carried out at a temperature of - 30°C or lower.

In another embodiment of the invention the low pH in step (d) is in the range of 1- 2 and in step (f) the pH is increased to a value in the range of 6 - 8.

In a further embodiment of the invention the mean MW of the HA in step (a) is 150 kDa and the mean MW of the HA in step (f) is 8 kDa.
In an example of this aspect of the invention, the lyophilization in step (a) is conducted at a temperature of - 50°C or lower, the lyophilization in step (b) is conducted at a temperature of -45 °C or lower, the pH in step (d) is 1.5 and the pH in step (f) is 6.5. In yet another embodiment of the invention, the method additionally comprises lyophilization of the stabilized colloidal aqueous suspension obtained in (f) together with added HA with a mean molecular weight of 1- 8 kDa, to obtain a free-flowing powder.
The obtained stabilized colloidal aqueous suspension containing a complex of amorphous HA and amorphous protein drug or free-flowing powder thereof according to the invention may be dispensed in dispensable dosage units for oral administration.
Alternatively, the obtained stabilized colloidal aqueous suspension containing a complex of amorphous HA and amorphous protein drug or a sterile solution thereof according to the invention may be dispensed in dosage units for parenteral administration.

Also in this aspect of the invention, the protein drug may be selected from the group consisting of insulin, glucagon-like peptide 1 (GLP1), human growth hormone (hGH), erythropoietin (EPO) and super oxide dismutase (SOD).

The "sufficient time period", with regard to freezing in steps a) and b) to produce amorphous products, will normally be from 10 hours to several days, such as 2 to 5 days. The term "hydrophobic surfaces are drawn to each other to form a complex" is used in this context to define bindings that are essentially of hydrophobic nature and influenced by long range forces and van der Waals forces between hydrophobic parts of a protein and the hydrophobic parts of HA.

The mixture of electrolytes and water in step c) will typically be aqueous solutions containing cations such as NH₄⁺, Na⁺, Ca²⁺, Mg²⁺, Zn²⁺, Fe²⁺, Fe³⁺ and anions such as sulfates, chloride, and acetates.

The reduction of the content of ions and of intramolecular bound water to a minimum accomplished in step e) is typically obtained by dialysis or diafiltration. The minimal amount of intramolecular bound water is within the complexes formed.

Free water is defined hereinbelow as non complex bound water contained in a solution or suspension. Consequently, the surrounding, free water content of the aqueous suspension to be lyophilized has no bearing on the intramolecular, bound water.

The concentration of complexed protein, such as complexed insulin, complexed erythropoietin, complexed growth hormone, or complexed super oxide dismutase, in the colloidal suspension is typically 0. 03 - 300 mg/mL.

In still another embodiment of the method according to the invention the obtained stabilized colloidal aqueous suspension of the complex of amorphous HA and reversibly compressed, amorphous protein drug or free-flowing powder thereof is dispensed in dispensable dosage units for oral administration.

The technology of producing a complex of hyaluronic acid and a biomolecule has been described in the European patent EP 1 231 926, the teaching of which is incorporated herein by reference. The storage life of said prior art complexes were indicated as 3 months at low, 2 - 8 °C, temperatures in said publication.

The orally deliverable pharmaceutical composition of the present invention has a storage life of at least 3 years at low temperatures of 2 - 8 °C. The water content of the suspensions may be expressed as a molarity of free water amounting to 0.78-1.1. This molarity represents an interval for obtaining a colloidal suspension, and gives a shelf life of the resulting product of at least 3 years at 4-15°C. Consquently, this interval is comprised by the term "minimum of water", as used herein. The molar ratio can be utilized irrespective of the scale of production.

In an embodiment of the invention, where the stabilized, colloidal aqueous suspension is lyophilized to a free-flowing powder, the storage life of the lyophilized free-flowing powder is 5 years at a temperature of 30°C. This lyophilized free-flowing powder has a considerably lower water content than the colloidal suspension, with the molarity of free water amounting to approximately 0.07 and lower. Surface-bound water is removed in the lyophilization process. However, enough droplets of water remain bound to the complexes by dipole moments, whereby the molecular mobility is retained and active sites of the protein drug are kept from deteriorating. The molecular mobility facilitates dissolution of the protein drug.

The lyophilized free-flowing powder enables a dramatically improved handling of a pharmaceutical composition comprising a protein drug such as insulin. It will not only be possible to administer insulin orally to a patient in need thereof but also to store the pharmaceutical composition comprising insulin at room temperature without the need of a refrigerator.

The content of water in the protein can be removed by sublimation or by spray drying resulting in a high-entropy form of the protein, the amorphous phase. The water molecules, by influence of the dipolar attraction forces, turn with great strength to form spots of water by self-association of water molecules. Sublimation, i.e. lyophilization, to an amorphous structure results in a more storage stable product compared to spray drying. The heat in spray drying tends to destroy the surface properties, the active sites of protein. A certain amount of water in the internal environment of the protein may however be necessary to prevent destruction of surface related molecule structures such as the -S-S- bridges upon spray drying.

Activity of superoxide dismutase with low content of water for *in vivo* use may be demonstrated *in vitro* by using vapour phase substrate. Such low content of water can be obtained for proteins/enzymes by freezing the enzyme in liquid nitrogen followed with lyophilization. Full activity of enzyme is obtained with a minimum of water that will be < 0.1 g per gram of protein.

Probably the structure of insulin goes from a hexameric towards a dimeric and a monomeric structure upon protonization. It is believed that the monomeric structure is the active form of insulin that activates the insulin receptor. In the present invention, Hyaluronan is stabilizing the monomeric form of insulin.

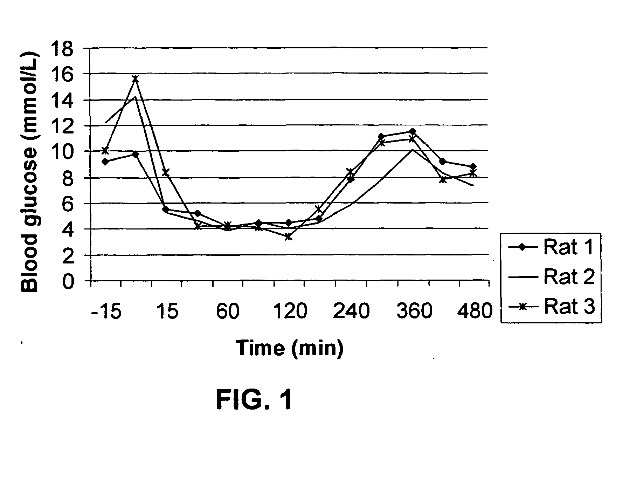

The acidity in the aqueous environments displaces the hydrophobic surfaces of the protonized protein and protonized Hyaluronan so that hydrophobic attraction forces are obtained. Loss of water reduces the strength of electronic interactions, the first acting forces in complexing the peptide, but both water dipole forces and the hydrophobic attraction force will cooperate in the complexed state, the protein is thought to have a small amount of water replaced with Hyaluronan. Both water dipole forces and the hydrophobic attraction force cooperate to complex the peptide structure and HA when water and ions are reduced by dialysis or diafiltration and related reducing processes. The energy of the amorphous state obtained by lyophilization of the protein contributes to a fast reaction of complexation with amorphous Hyaluronan, but also to build in energy in the complex necessary in the absorption processes for e.g. insulin delivery to the arterial blood. Another feature is the stability of the Hyaluronan insulin complex against enzyme degradation. We have not noted any degradation of the complexed insulin expressed as loss of activity in glucose decrements upon repeated subcutaneous injections nor upon oral application of complexed insulin down to the cardia in STZ-rats. The more than two years physicochemical stability of the colloidal suspension is explained by an added extra amount of Hyaluronan which contributes to sterical hindering of physical settling and development of sediments in a container of the colloidal suspension of the complex. The stability of bioactivity for the Hyaluronan insulin complex after two years of storage is shown in experiments with GK rats with inherited diabetes and results in glucose decrement (See FIGs. 1 -3). The *in vivo* activity of the insulin complex is due to the amorphous state of the complex. The lyophlization at low temperature, - 30°C or less, will contribute to retain the activity of the protein.

In the complexation procedure it is shown that water and ions are removed but the functionality of the complex of Hyaluronan and protein, e.g. insulin, is retained *in vivo.* Complete removal of water should not rule out the possibility of protein activity, but the limit for removal of water is associated with onset of intermolecular motions. The active site of the protein should have a minimal hydration level and is a limit for a lyophilized protein. In the complexation procedure it is shown that the functionality of the complex of Hyaluronan and protein, e.g. insulin is retained *in vivo* even though water and ions are removed. Complete removal of water is not desirable, thus a minimum of water should be present in order to retain the functionality of the active site.

The invention will now be illustrated with reference to experiments and examples as well as the drawings, but it should be understood that the invention is not limited to any disclosed specific details and that the scope of protection is defined by the appended claims.

### Short description of the drawings

**FIG. 1** is a diagram that shows blood glucose levels (mmol/L) plotted against time (min). Subcutaneous administration of complexed insulin at time-point 0 to rats with inherited diabetes (GK-rats). The complexed insulin was stored for two years and was prepared from amorphous insulin and amorphous HA. The injection was a subcutaneous injection of 110 µg complexed insulin contained in an isotionic solution (308 mOsmol/dm³) stored for two years.

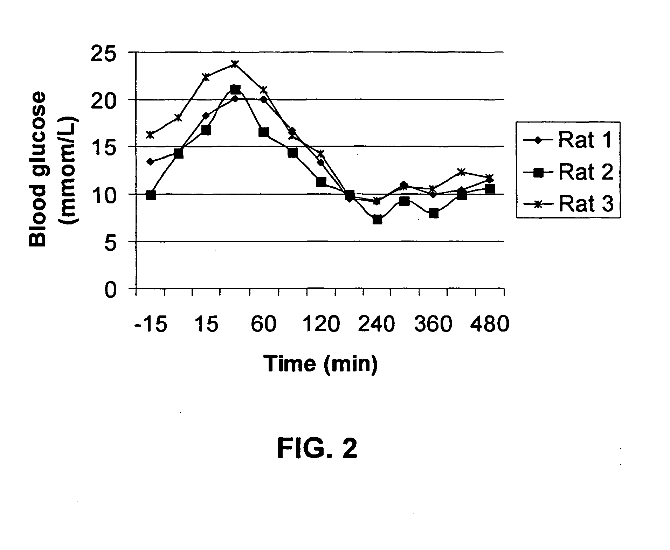

**FIG. 2** is a diagram that shows blood glucose levels (mmol/L) plotted against time (min). Oral administration of complexed insulin at time-point 0 to rats with inherited diabetes (GK-rats). The complexed insulin was stored for two years and was prepared from amorphous insulin and amorphous HA. 3.7 mg complexed insulin contained in a hypotonic solution (150 mOsmol/dm³) stored for two years was administered orally.

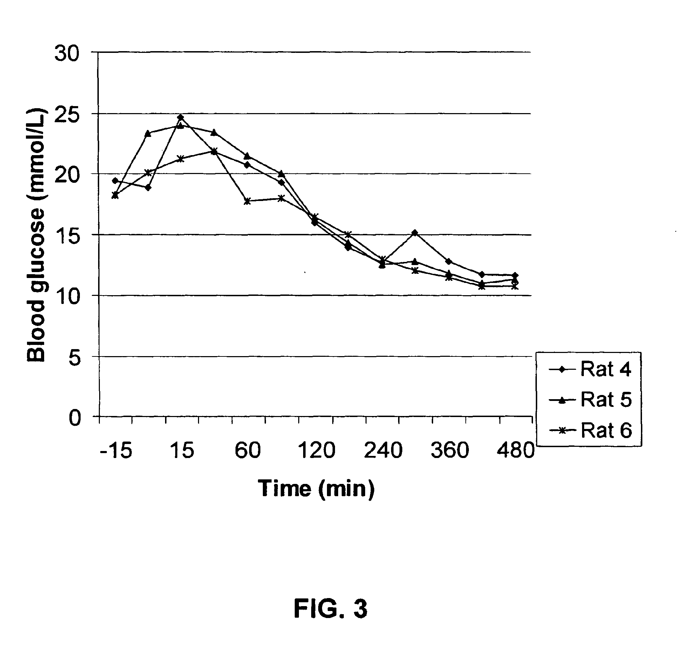

**FIG. 3** is a diagram that shows blood glucose levels (mmol/L) plotted against time (min). Oral administration of complexed insulin at time-point 0 to rats with inherited diabetes (GK-rats). The complexed insulin was stored for two years and was prepared from amorphous insulin and amorphous HA. 3.7 mg complexed insulin contained in a hypotonic solution (150 mOsmol/dm³) stored for two years was administered orally.

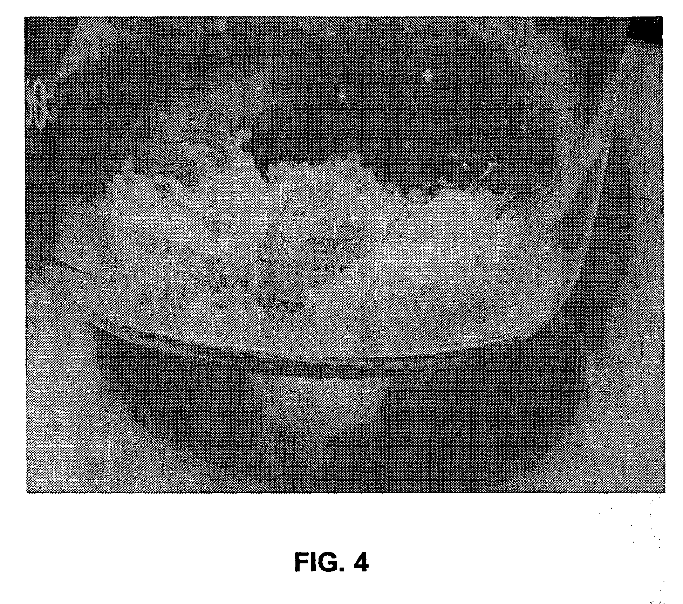

**FIG. 4** shows a photograph of crystalline Hyaluronan 30 kDa in a beaker.

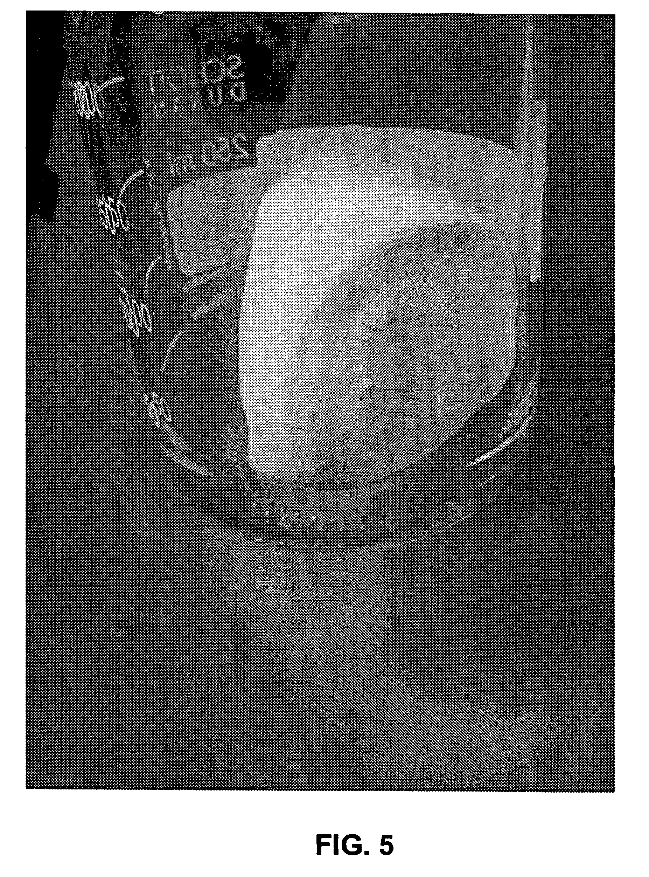

**FIG. 5** shows a photograph of cotton-like amorphous Hyaluronan 150 kDa in a beaker.

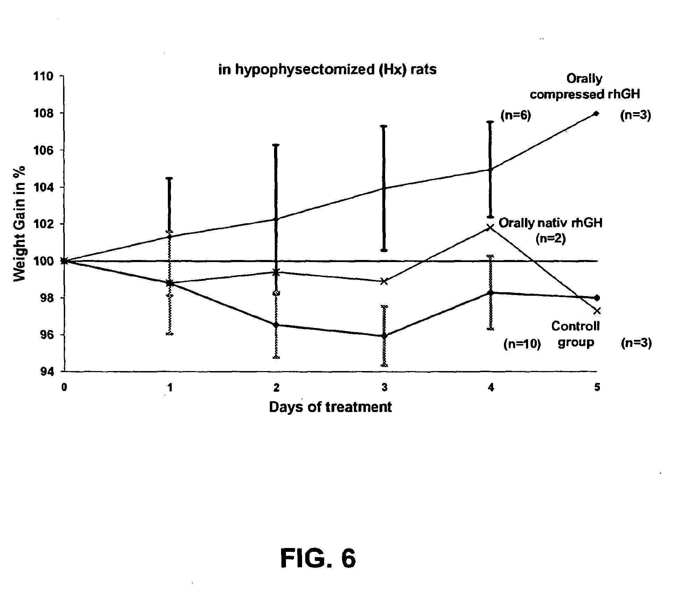

**FIG. 6** is a diagram that shows body weight (BW) gain in hypophysectomized (H-x) rats in % plotted against days of treatment with orally administered complexed rhGH 1.5 (n=3) and 0.5mg/ml (n=3), prepared from amorphous rhGH and amorphous hyaluronan, and compared with oral rhGH 1.5 mg/ml (n=2) and a control group (n=10 ,n=3).

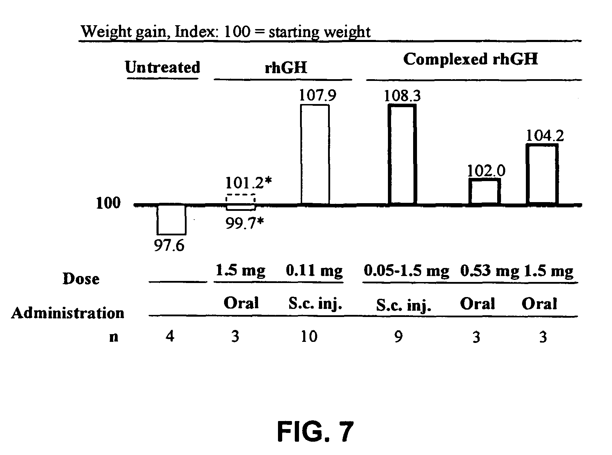

**FIG. 7** is a diagram that shows bodyweight (BW) gain as percentage of initial BW in hypophysectomized (H-x) rats orally and subcutaneously treated with complexed rhGH (high and low dose) prepared from amorphous rhGH and amorphous Hyaluronan, a control group consisting of untreated H-x-rats and BW gain of orally and subcutaneously administerad rhGH.
   * Data indicates that one rat was insufficiently ectomized; 99.7 corresponds to removal of data for this rat.

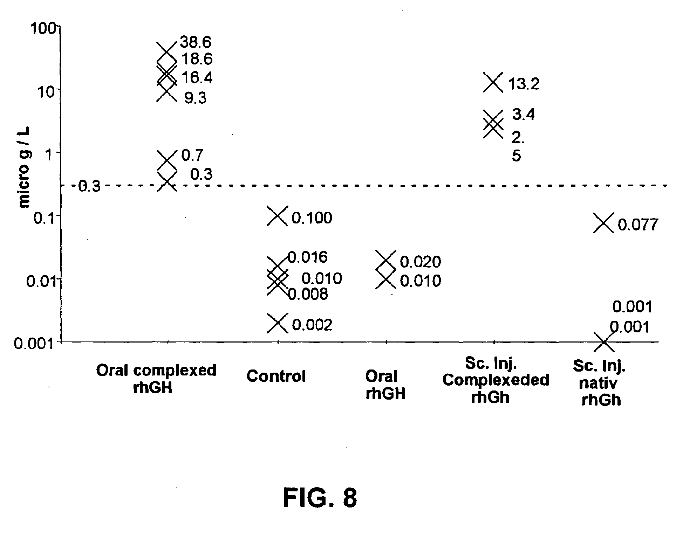

**FIG. 8** is a diagram that shows serum levels in microgram /L of rhGH in hypophysectomized (H-x) rats after oral and subcutaneous administration of complexed rhGH prepared from amorphous rhGH and amorphous Hyaluronan, and of rhGH and untreated H-x rats (control group) determined by Auto Delfia®. Value below 0.3 µg/L is under the detections level and can be omitted.

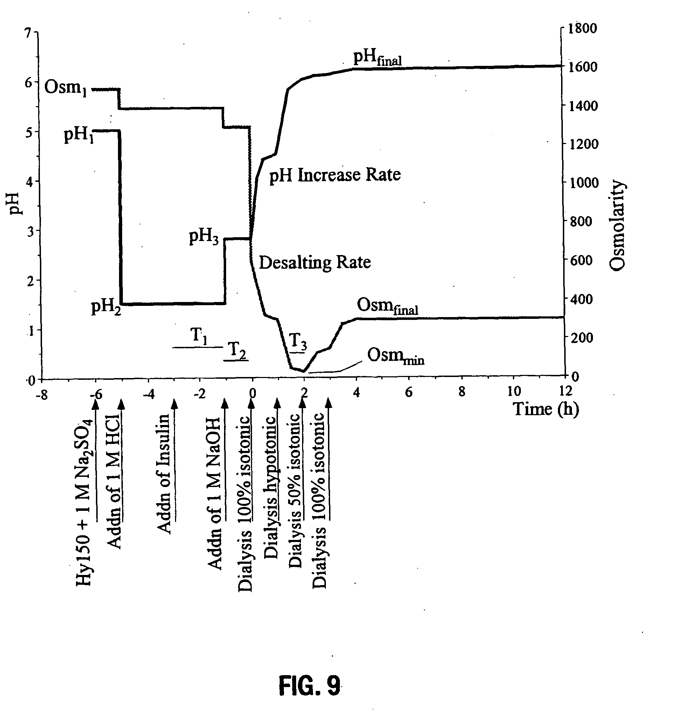

**FIG. 9** is a figure showing the pH and osmolarity during the desalting (dialysis) steps of manufacture of complexed insulin for injection purposes.

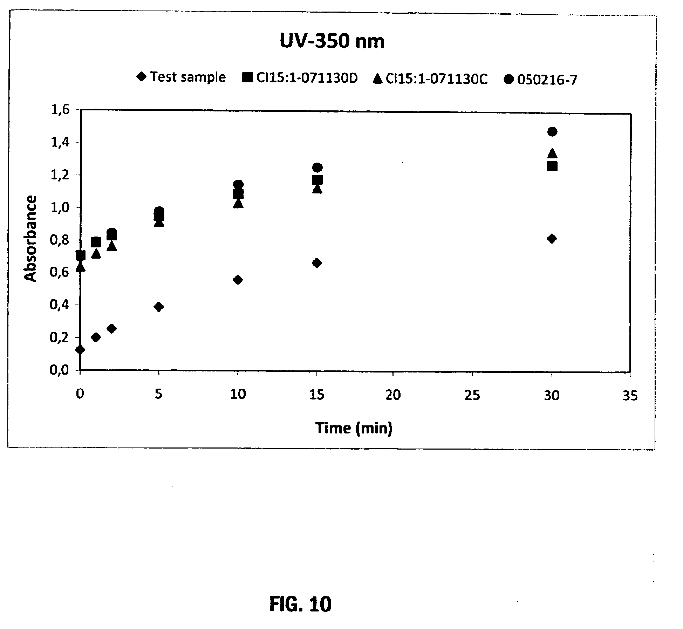

**FIG. 10** is a figure showing the absorbance of three suspensions, one of which has been stored and two of which are freshly prepared.

### EXAMPLES

### Example 1: Lyophhilization of Hyaluronic acid to produce amorphous Hyaluronic acid.

In anticipation of lyophilization, the Hyaluronic acid is frozen on dry ice and ethanol. The integrity of the product is ensured by protecting the ice slush from the surrounding water and air by use of an oxygen-free atmosphere, an inert gas atmosphere, such as argon gas atmosphere, in a closed environment. Such oxygen-free atmosphere is used throughout the lyophilization process. A certain setting of the product is caused by raising the temperature of the ice somewhat. The lyophilization is effected at a negative pressure of at least 0.8 bar and a temperature below -30 °C during 3-4 days. The lyophilization cake is observed by way of ocular inspection, to make sure it has not collapsed. The lyophilization process is concluded by supplying additional inert gas. By using said method, secondary drying is superfluous.

| | |
|---|---|
| Starting material Sodium Hyaluronan | |
| Mean molecular weight | 8 x 10⁶ Da |
| Water content | 8.1 % w/w |
| Protein content | < 0.5 mg/g |
| Content of sodium hyaluronan | 92 % w/w |

### Dissolution

2.51 mg of Sodium Hyaluronan is dissolved in 500 ml oxygen free distilled water, whereupon a supplement of 0.05 % sodium azide may be added to a final volume of 1000 ml in a closed bottle, which has been purged and filled with inert gas. The contents of the bottle were stirred during more than 20 hours.

### Hydrolysis of Hyaluronan 8 000 kDa to hyaluronic acid 150 kDa (mean MW)

16 ml of 1 M Hydrochloric acid is added, whereby the bottle is purged with inert gas and the contents are stirred during 2 hours. The pH of the contents should be less than 1 and continuously checked. The solution is neutralized to pH 7.0 by addition of 0.5 M sodium hydroxide. Thereafter, 25 mg of sodium azide may be added.

### Dialysis procedure

The neutralized solution with pH 7 is transferred to a 1 liter beaker, whereby the solution and the bottle are filled with inert gas and free radicals are removed. A dialysis tube, with a cut of 12-14 x 10³ Dalton, is immersed into the solution. The conductivity is measured, and the time sufficient dialysis is set to a dialysis interval ranging from 3µS/cm to 0.8 µS/cm. The time to accomplish a sufficient dialysis is approximately 4 days.

### Lyophilization

600 ml of the above solution was dispensed into three different bottles purged with inert gas. The solutions were cooled to a temperature below -60°C, whereupon the temperature was kept between - 55 and -58 °C during evacuation. The initial evacuation pressure is 8 mBar, or 0.4 atm, during a time period of at least approximately 48 hours. The final pressure during evacuation should not exceed 3.2 mBar or be ≤ 0.06 atm. The cotton-like Hyaluronic acid with a weight not exceeding 0.67 g is stored in a closed container containing inert gas, at a temperature not exceeding - 20 °C.
See FIG. 5 illustrating cotton-like amorphous Hyaluronan 150 kDa

### Example 2. Preparation of amorphous Human growth hormone.

Human growth hormone (rhGH) is diluted in a lysine buffer to a solution with a pH of approximately 7.15 and a concentration of approximately 108 I.U/ml.

At this concentration of rhGH or higher, the rhGH solution by it self formulates to contain a functional buffer. The concentrated solution is purged with inert gas, to protect the disulphide bonds. The solution in an amorphous state contains a minimum of water, i.e. 0.01 g water / g of rhGH. However, lower water content disrupts the disulphide bonds. The primary and secondary structure needs to be retained. The lyophilization process thus proceeds during an extended period of time, at a low temperature, whereby sublimation occurs.

The solution is loaded at a temperature of 20°C. Thereafter, the temperature is gradually lowered to -10°C during 30 minutes. This freezing continues at -10°C during 1.5 hours, whereupon the temperature is decreased to -45°C during 1.5 hours. Lyophilization at -45°C was then executed for 15 hours. The primary drying at - 30°C is executed at a pressure of 0,1 bar, whereupon the temperature is increased to +25°C during 2 hours, followed by an optional secondary drying at +25°C during 5 hours. The secondary drying is not necessary using this protocol. As an alternative, spray drying may be used. However, whereas the water content should be as low as possible, care must be taken not to destroy the disulphide bonds at higher temperature due to burning.

### Example 3. Preparation of a complex of amorphous Hyaloruonic acid and amorphous Human growth hormone.

The lyophilized rhGH in amorphous state is complexed with amorphous Hyaluronan. 15 mg amorphous Hyaluronan is dissolved in 1 2 mL of 1 M Sodium Sulphate (Na₂S0₄) - solution and pH is adjusted to 1.51 with 1 M HCl. 22 mg amorphous rhGh is dissolved in the Hyaluronan solution. 1M HCl is added to obtain a transparent solution. The obtained dispersion is purged with inert gas and is transferred into dialysis bags with a size of 3 ml and dialyzed with trometanol buffer pH 6.5 in three occasion with a portion of about 1.2 L. A precise measured volume in dialyze bags obtained is brought into an injection vial purged with inert gas and capsulated. Samples are taken for assay of contents, particle size, identification with size exclusion chromatography and sterility test.

The complexed rhGH is after approval, storage tested and used in preclinical studies. Physicochemical testing expressed storage stability in excess of 36 months at a temperature of 2 °C -8 °C. After 36 months of storage, the rhGH preparation showed subcutaneous as well as oral effect (See FIGs. 6, 7 and 8). This indicates that a minimal specific molar ratio of buffer to protein is sufficient to provide optimal stabilization against aggregation and degradation, during storage of lyophilized rhGH. The reduction in aggregation and deamidation during storage correlated directly with inhibition of unfolding during lyophilization. Thus, it appears that the protein must retain its primary and secondary structure during lyophilization to assure storage stability of the lyophilized solid. This is accomplished by selecting an appropriately high concentration (molar ratio) of protein to the particular buffer. The buffer should be in a minimal concentration. Crystal formation should be avoided, to reduce mobility of the protein structure.

### Example 4. Preparation of amorphous insulin.

Lyophilization of insulin is conducted substantially in accordance with example 2. The stabilization of insulin folding after compression is due to the Hyaluronan complexation that arranges and stabilizes the folding in insulin.

### Example 5. Preparation of a complex of amorphous Hyaloruonic acid and compressed amorphous Insulin.

The amourphous Hyaluronan prepared in Example 1 is weighed in and 6.5 mL of 1.0 M Na₂SO₄ and a small magnetic stirring bar is placed in the bottle and gentle stirring is applied. During all processing and manufacturing all containers and all solutions are purged with inert gas. All containers are also closed under inert gas.

The Hyaluronan bottle is opened, using a micropincers. Start time and time when a clear solution is obtained is noted. pH is adjusted to 1.5-1.6 using 1.0 M HCl and 1.0 M NaOH. When approaching pH 1.5, the viscosity should decrease. Amorphous Insulin is added by placing the injection bottle containing insulin on top of the bottle containing the HA. pH is measured and noted. Gentle magnetic stirring is performed for 1 hour. pH is adjusted to 2.0-3.0 using 1.0 M NaOH and 1.0 M HCl. When approaching pH 2.0, the viscosity should increase. Final pH, added amount NaOH and HCl are noted. Gentle magnetic stirring is performed for 2 hours. Note start and finish time. Note ocular appearance (opacity, viscosity). Cut dialysis tubing 10 cm x 3. Dialysis is started in 10 mM Tris buffer, pH 6.5 or use diafiltration. For the dialysis procedure. Dispense the dispersion into dialysis bags, using a micropipette. Leave 0.5 cm of the tubing outside the closure on one side. Seal with labelled dialysis tubing closures. Introduce the bags to the dialysis buffer. Note the time when all bags have been placed in the buffer. The dialysis buffer is changed as described below. Times for changing of the medium are noted. pH is measured after each dialysis period and the dispersions and bags are inspected (dispersion: e.g., transparent, cloudy, heavy precipitate, light precipitate, stripes of white precipitate; bags: expanded or loose)

Dialysis is performed with the following solutions.
2 L 100% Tris Buffer (TB) with 0.9% NaCl for one hour
2 L 100% TB for one hour
2 L 100% TB for one hour
2 L 100% TB for 18-24 hours
2 L 100% TB for 20-28 hours
2 L 100% TB for 18-20 hours

For oral dispersion the dialysis procedure is stopped at this point.

For parenteral preparations 2 L 100% TB for 18-20 hours is exchanged with 2 L of 100% Tris Buffer (TB) with 0.9% NaCl and the dialysis is continued to obtain istonicity of the solutions with complexed insulin.

The dialysis bags are opened and their contents transferred to an injection vial, using a micropipette. The total volume is noted. pH of the final product is measured and noted.

### Example 6. Amount of free water in a pharmaceutical preparation with complexed insulin.

An example of a "minimum of water" in a protein complexed preparation with full biological effect after oral administration and which is physicochemical stable for three years is given below.

The complexed insulin composition contains Insulin (Mw 5812), Hyaluronan (Mw 160 000) and holds a minimum of water in the final preparation in 8 different preparations with a molar ratio Insulin : Hyaluronan of 15:1 and with a water content of 14-20 ml. The formulation is prepared from 45 mg amorphous insulin and 82 mg amorphous Hyaluron. Eight preparations containing this amount of insulin and Hyaluronan (in a molar ratio 15:1) is complexed in a free radical reduced aqueous suspension resulting in a final water content in the range of 14 -20 ml., which gives the limit of water for this formulation to obtain 3 years stability (3 years shelf life) of the suspension of the Hyaluronan-insulin complex, with retained, full biological activity, as demonstrated in GK rats.

This minimum of water in the final preparation is obtained by desalting the free water during complexation at an almost zero concentration of ions, and by limiting the water content in stabilization of the protein complex in the dialysis procedure (dialysis process). These processes are illustrated in figure 8 below. The minimum of free water and the ratios between free water and insulin and hyaluronan, respectively, are for a long term storage suspension (4 years at a temperature of 4-15 °C) of a complexed protein (insulin) expressed by the following molar ranges;

| | Insulin | Hyaluronan | Water |
|---|---|---|---|
| Mw | 5812 | 160000 | 18 |
| Molarity | 51 - 77 x 10⁻⁷ | 5.1x 10⁻⁷ | 0.78-1.1 |

The desalting steps during the manufacture of complexed insulin are graphically depicted in Fig. 9, wherein an isotonic suspension for injection purposes is prepared.. Here, the dialysis continues for approximately 60 hours, with exchange of the medium every 24 hours. In the present example, an isotonic solution was initially used. Thereafter using hypotonic solutions during dialysis, an osmolarity of close to zero is achieved at 2 h. Subsequently, dialysis is again performed with isotonic solutions. The term "50 % isotonic" as used in Fig. 9 denotes a dialysis solution containing 50 % isotionic solution and 50 % hypotonic solution. During the dialysis steps, the amount of water is gradually reduced. After the dialysis, the osmolarity of the hypotonic solution is close to zero. The decrease in number of ions has reduced the amount of free water, which in turn improves the stability of the complex. The final osmolarity of 300 Osm is suitable for injection purposes. For suspensions that are to be lyophilized, the final dialysis steps are instead performed with hypotonic solutions.

The free water content in the pharmaceutical preparations is finally limited by adding an excess amount of hyaluronic acid, which may act as a scavenger to assure storage stability of the colloidal dispersion and of the lyophilized free flowing powder in capsule.

### Example 7 - Physicochemical stability of suspensions of complexed insulin

Stability of the stabilized, colloidal aqueous suspensions according to the invention is assessed by use of several methods, using the criteria below. For complexes to be utilizable in the formation of a lyophilized free-flowing powder, the suspensions shall be homogenous and not express any signs of cake formation.

### Homogeneity

Homogeneity of batches is determined by visual inspection and turbidimetric measurements of the suspensions, whereby the time-dependent homogeneity of the suspension is determined.

### Visual inspection of scattered light

Visual inspection of scattered light in the suspension of complexed insulin is attributed a value from 1 to 4 according to pharmaceutical praxis as previously described in Pharmacopea. Upon shaking a suspension of the invention, the suspension is easily dispersed, whereby its appearance is transformed from a opaque appearance on the bottom of the vial to that of a homogenous colloidal suspension with Tyndall properties. No flocculation of the particles is observed and the sedimentation occurs gradually. The homogeneity is determined within 3 minutes.

### Turbidmetric measurements

The turbidity (light scattering) is monitored at 360, 540 and, in one case, 820 nm. As the light scattering is more prominent at lower wavelengths, the sensitivity of the method is best monitored at 360 nm. For the suspension to be viewed as homogenous, the absorbance at 360 nm should be in the interval of 2.0-0.9, and should ideally stay within the range of 1.1-0.85 during a time period of 10 minutes.

Visual inspection of light-scattering particles has been compared with turbid metric measurements. The obtained relationship between visual inspection (1-4 as described above) and light scattering is given in Table 1.

**Table 1. Visual inspection of Complexed Insulin is read out from 1 to 4 and is equal to the given intervals of turbidimetric read out on a standard spectrophotometer at 360 nm.**

| Value of turbidity of visually inspected suspension of complexed insulin | Turbidimetric light-scattering at 360 nm |
|---|---|
| 1 | 1.4-2.0 |
| 2 | 0.9-1.4 |
| 3 | 0.6-0.9 |
| 4 | 0.3-0.6 |

### Visual particles

The number of particles seen visually (usually with a size ≥ 5 µm) in homogenous colloidal suspension does not exceed 10 per 4 mL (2.5 per ml) in suspensions of the invention.

**Table 2. Stability measurements**

| Preparation date of Complexed Insulin YY,MM,DD/ Batch # | Turbidity Visual Inspection¹ | | Absorbance at 360 nm | | Visual Particles >5µm² (# of particles/ml) | | Storage time of Complexed Insulin (months) |
|---|---|---|---|---|---|---|---|
| | At Preparation date | 05 May 2008 | At Preparation date | 05 May 2008 | At Preparation date | 05 May 2008 | |
| 040823 | 3 | 3 | 0.8 | 0.8 | 1.5 | 1.5 | 44 |
| 040906 | 2 | 2 | - | 0.9 | 1.5 | 1.5 | 43 |
| 041025 | 2 | 2 | 0.9 | 0.9 | > 1 | 1.0 | 42 |
| 050118/2 | 4 | 4 | 0.5 | 0.6 | 1.5 | 1.5 | 39 |
| 050126/3 | > 1 | 1 | 2.4 | 2.4 | 0 | 0 | 39 |
| 050126/4 | 3 3 | | 0.8 | 0.6 | 1.5 | 1.5 | 38 |
| 050216/7 | 1-2 | 1 | 2.0 | 2.0 | 0 | 0 | 38 |
| 050216/8 | ≥ 1 | 1 | 2.0 | 2.0 | 0 | 0 | 38 |

| | | | | | | | |
|---|---|---|---|---|---|---|---|
| ¹ Ranking scale, range 1-4, where 1 is high turbidity and 4 is low turbidity. ² Estimated number of particles found in 10 ml suspension. | | | | | | | |

At time of preparation, the suspensions had a pH in the range of 6.4-6.7. The pH was retained in this interval during the time of storage (38-44 months).

### Caking

Particles growing into a one-unit or multi-unit cake of particles during storage is defined as caking. When shaking such a suspension, one or more cakes appear. No caking is visually observed in the complexed insulin compositions of the invention.

### Example 8

The storage capability of complexes manufactured in an inert gas, free radical reduced environment can be illustrated by the measurement of free radicals in suspensions of the invention. Here, an assessment of stability is carried out by firstly measuring the content of free radicals in two newly prepared suspensions,of complexed insulin (C115:1-071130C and C115:1-071130D) prepared in free radical reduced environment and comparing them with the radical content of a similarly prepared suspension of complexed insulin (050216-7) stored during 2 years and 9 months (33 months) at a temperature of 5-8 °C..

Storage stability according to the invention is clearly shown by the comparative experiment, wherein use is made of a chemical reaction as described by Bloomfield in "the spectrophotometric determination of hydroperoxide and peroxide in a lipid pharmaceutical product by flow injection analysis, Analyst 1999, 124, 1865-1871.

FIG. 10 illustrates that the content of free radicals in the compared solutions remains the same. This shows that long-term storage (33 months) of a free radical reduced suspension according to the invention does not increase the amount of free radicals present therein, compared with newly prepared preparations. Free radicals are measured as the absorbance at 350 nm from free iodine, transferred from iodide.
- Blank solution:: 4g acetic acid diluted to 100ml with 80% 2-propanol:20% Milli-Q
- Potassium iodide solution:: 10g Kl dissolved in 15ml Milli-Q water and diluted to 100ml with 2-propanol
- Test sample:: 2.5ml Blank solution
0.5ml KI-solution
- Sample:: 2.0ml Blank solution
0.5ml Kl solution
0.5ml Sample (CI15:1-071130D; CI15:1-071130C and 050216-
7,, respectively)

UV-Measurement, at
350nm

**Table 3. Storage stability**

| Absorbance | | | | |
|---|---|---|---|---|
| Time | | | | |
| (min.) | Test sample | CI15:1-071130D | CI15:1-071130C | 050216-7 |
| 0 | 0.127 | 0.705 | 0.636 | 0.697 |
| 1 | 0.202 | 0.786 | 0.717 | 0.791 |
| 2 | 0.256 | 0.828 | 0.764 | 0.847 |
| 5 | 0.390 | 0.952 | 0.915 | 0.980 |
| 10 | 0.559 | 1.088 | 1.032 | 1.146 |
| 15 | 0.667 | 1.178 | 1.125 | 1.254 |
| 30 | 0.827 | 1.273 | 1.355 | 1.486 |
| Abs/time | 0.023 | 0.018 | 0.023 | 0.025 |

| | | | | |
|---|---|---|---|---|
| Notes: CI15:1-071130D: Freshly prepared suspension of complexed insulin CI15:1-071130C: Freshly prepared suspension of complexed insulin 050216-7: Stored (33 months) suspension of complexed insulin | | | | |

### Example 9

### Pharmaceutical compositions

### Solutions for Injection

Injections are sterile solutions, emulsions or suspensions. They are prepared by dissolving, emulsifying or suspending the active substance(s) and any added excipients in water for injections, in a suitable, sterile non-aqueous liquid or in a mixture of these vehicles. Solutions for injection, examined under suitable conditions of visibility, are clear and practically free from particles. Suspensions for injection may show a sediment which is readily dispersed on shaking to give a suspension which remains sufficiently stable to enable the correct dose to be withdrawn.

In the manufacture of injections containing dispersed particles, measures are taken to ensure a suitable and controlled particle size with regard to the intended use. The volume of the injection in a single-dose container is sufficient to permit the withdrawal and administration of the nominal dose using a normal technique.

Complexed insulin in a concentration of 3 mg/ml for parenteral use is contained in a 10 ml multidose container, whereby precautions are accounted for regarding its administration and more particularly for its storage between successive withdrawals. The example refers to a complex of insulin and hyaluronan (15:1), in the form of a suspension; 3.0 mg/ml. The batch size amounts to 45 mg. Complexed insulin in a suspension of 3 mg/mL in a 1mL single container (an ampoule) or in a 10 mL single- or multidose container for administration through a feeding tube is prepared.

The suspension is shaken before use, and stored at a temperature of 5-8°C, for a time of 36 months. The contents of an opened container should be used within 10 days, and stored at a temperature of 5-8°C between successive withdrawals.

Complexed insulin is a complex of human recombinant (rh) Insulin and Hyaluronan, mean MW 150 kDa, in a molar ratio of 9 -20:1 formulated with sodium chloride to a sterile isotonic aqueous suspension; for oral use without sodium chloride. Settled sediment is readily dispersed on shaking to give a homogenous suspension which remains stable to enable the correct dose to be withdrawn.

The suspension for parenteral use and the procedure are aseptically performed. Finally the dispersion with complexed insulin is aseptically dispensed in Type I glass containers suitable for parenteral use. Glass containers are tested in accordance with the European Pharmacopoeia.

The size of dispersed particles is measured with DynaPro 801. Agglomerate 200 microns containing particles less than 20 nm.

*Type* / *glass containers for parenteral use, or single-dose containers,* are filled with a volume of 1.5 ± 0.5 ml. *The multidose containers are* filled with a volume of the 11.5 ± 1.5 ml, and sealed with rubber closures for containers for aqueous parenteral preparations, for powders and for freeze-dried powders, in accordance with European Pharmacopoeia .

No cysteic acid originating from the cleavage and oxidation of -S-S- bridges was detected during amino acid analysis. The molar ratio between insulin and hyaluronan is 9 -20:1. The content of complexed insulin was assayed to be native insulin 2.5 ± 0.5 mg/ml. The concentration of hyaluronan was 3.5-6.6 mg/ml.

The osmolarity is 290-320 mmol/kg for injection purposes 180 mmol/kg and the pH 5.0-7.0.

Biological assay was performed to determine the concentration of blood glucose after subcutaneous injection of the composition in diabetic rats. For STZ diabetic rat, the decrement should be ≥ 15 mM within 1 hour. The assay was performed in accordance with Jederstrom et al., Pharm Res. 2004, 21(11):2040-7.

### Suspension for oral use

Oral administration in a single dos container or a multidose container equipped with a metered dispensable device.

### Gastro-resistant hard shell capsules

Complexed insulin in gastro-resistant capsules containing 3 mg of complexed insulin obtained by comlexation of amorphous insulin and amorphous hyaluronan and determined as native insulin, intended for use by oral administration, is prepared. The capsules are solid preparations with hard shells provided with a gastro-resistant shell (i.e. enteric capsules).

The gastro-resistant capsule is prepared as a delayed-release capsule to resist the gastric fluids and to release complexed insulin in the intestinal fluid and is prepared by filling the capsules with the free flowing powder of the invention. The free-flowing powder releases complexed insulin within 60 seconds after leaving the stomach.

TESTS Gastro-resistant soft shell capsules and hard shell capsules diluted with water 1+1

The amino acid analysis showed:
- No cysteic acid originating from the cleavage and oxidation of -S-S- bridges was detected during amino acid analysis;
- The molar ratio between insulin and hyaluronan was 9-20:1
- The content of complexed insulin assayed native insulin 2.5 ± 0.5 mg/ml.

The concentration of hyaluronan was 3.5-6.6 mg/ml,

## Claims

1. A stabilized, colloidal free radical reduced aqueous suspension containing a complex of amorphous Hyaluronic acid (HA) with a mean molecular weight (mean MW) in the range of 80 to 400 kDa and an amorphous protein drug, and an excess amount of HA to minimize the water content.

2. The stabilized, colloidal aqueous suspension according to claim 1, wherein the HA of the excess amount of HA has a mean MW in the range of 6 - 15 kDa.

3. The stabilized, colloidal aqueous suspension according to claim 1 or 2, wherein the suspension additionally comprises HA with a mean MW in the range of 1 - 8 kDa and is in lyophilized free-flowing powder form.

4. The stabilized, colloidal aqueous suspension according to any one of claims 1-3, wherein the protein drug is selected from the group consisting of insulin, glucagon-like peptide 1 (GLP1), human growth hormone (hGH), erythropoietin (EPO) and super oxide dismutase (SOD).

5. A pharmaceutical composition comprising a stabilized, colloidal aqueous suspension according to any one of claims 1 - 4, and a pharmaceutically acceptable carrier.

6. The pharmaceutical composition according to claim 5, wherein the pharmaceutically acceptable carrier is an orally dispensable dosing device for oral administration of the colloidal aqueous suspension.

7. The pharmaceutical composition according to claim 5, wherein the pharmaceutically acceptable carrier is an orally deliverable capsule.

8. The pharmaceutical composition according to claim 7, wherein the capsule is designed for enteric delivery of the composition.

9. The pharmaceutical composition according to claim 5, wherein the pharmaceutically acceptable carrier is a dosing device for parenteral administration of the colloidal aqueous suspension.

10. A method of producing a stabilized, colloidal free radical reduced aqueous suspension of a complex of Hyaluronic acid (HA) and a protein drug, comprising carrying out the following steps under inert conditions:
(a) lyophilizing an aqueous solution of HA with a mean molecular weight (mean MW) in the range of 80 and 400 kDa and maintaining the lyophilization during a sufficient time period, to obtain the HA in amorphous form,
(b) lyophilizing an aqueous solution of the protein drug and maintaining the lyophilization during a sufficient time period, to obtain the protein drug in amorphous form,
(c) admixing the lyophilized, amorphous HA with a mixture of electrolytes and water, whereby electrical double layers are obtained on the HA of the resulting homogenous mixture,
(d) adding the lyophilized, amorphous protein drug at a low pH during a time period that is sufficiently short not to denature the protein, whereby electrical double layers are obtained on the amorphous protein drug,
(e) reducing the content of water and ions to a minimum, whereby hydrophobic surfaces are drawn to each other to form a complex of the amorphous HA and the amorphous protein drug; and whereby the pH of the complex increases, and
(f) adding an excess amount of HA with a mean MW in the range of 6 - 15 kDa in the minimum of water to produce the stabilized colloidal suspension containing the complex of the amorphous HA and the amorphous protein drug.

11. The method according to claim 10, wherein the lyophilization in step (a) is carried at a temperature of - 40ºC or lower.

12. The method according to claim 10, wherein the lyophilization in step (b) is carried at a temperature of - 30ºC or lower.

13. The method according to claim 10, wherein the low pH in step (d) is in the range of 1- 2 and in step (f) the pH is increased to a value in the range of 6 - 8.

14. The method according to claim 10, wherein the mean MW of the HA in step (a) is 150 kDa and the mean MW of the HA in step (f) is 8 kDa.

15. The method according to claim 10, wherein the lyophilization in step (a) is conducted at a temperature of - 50ºC or lower, the lyophilization in step (b) is conducted at a temperature of -45 ºC or lower, the pH in step (d) is 1.5 and the pH in step (f) is 6.5.

16. The method according to any one of claims claim 10, wherein the method additionally comprises lyophilization of the stabilized colloidal aqueous suspension obtained in (f) together with added HA with a mean molecular weight of 1- 8 kDa, to obtain a free-flowing powder.

17. The method according to claim 10 or 16, wherein the obtained stabilized colloidal aqueous suspension containing a complex of amorphous HA and amorphous protein drug or free-flowing powder thereof is dispensed in dispensable dosage units for oral administration.

18. The method according to any one of claims claim 10 -13, wherein the obtained stabilized colloidal aqueous suspension containing a complex of amorphous HA and amorphous protein drug or a sterile solution thereof is dispensed in dosage units for parenteral administration.

19. The method according to any one of claims claim 10 -18, wherein the protein drug is selected from the group consisting of insulin, glucagon-like peptide 1(GLP1), human growth hormone (hGH), erythropoietin (EPO) and super oxide dismutase (SOD).

## Patentansprüche

1. Stabilisierte collidale radikalreduzierte wässrige Suspension, enthaltend einen Komplex aus amorpher Hyaluronsäure (HS) mit einem mittleren Molekulargewicht (mittlerem MG) im Bereich von 80 bis 400 kDa und einem amorphen Proteinarzneistoff sowie einen Überschuss an HS zwecks Minimierung des Wassergehalts.

2. Stabilisierte colloidale wässrige Suspension nach Anspruch 1, wobei die HS des Überschusses an HS ein mittleres MG im Bereich von 6-15 kDa aufweist.

3. Stabilisierte colloidale wässrige Suspension nach Anspruch 1 oder 2, wobei die Suspension zusätzlich HS mit einem mittleren MG im Bereich von 1-8 kDa umfasst und in Form eines lyophilisierten freifließenden Pulvers vorliegt.

4. Stabilisierte colloidale wässrige Suspension nach einem der Ansprüche 1-3, wobei der Proteinarzneistoff aus der Gruppe bestehend aus Insulin, Glucagon-Like Peptide 1 (GLP1), menschlichem Wachstumshormon (hGH), Erythropoietin (EPO) und Superoxiddismutase (SOD) stammt.

5. Pharmazeutische Zusammensetzung, umfassend eine stabilisierte colloidale wässrige Suspension nach einem der Ansprüche 1-4 sowie einen pharmazeutisch unbedenklichen Träger.

6. Pharmazeutische Zusammensetzung nach Anspruch 5, wobei es sich bei dem pharmazeutisch unbedenklichen Träger um eine oral abgebbare Dosiereinheit für die orale Verabreichung der colloidalen wässrigen Suspension handelt.

7. Pharmazeutische Zusammensetzung nach Anspruch 5, wobei es sich bei dem pharmazeutisch unbedenklichen Träger um eine oral verabreichbare Kapsel handelt.

8. Pharmazeutische Zusammensetzung nach Anspruch 7, wobei die Kapsel für die enterale Abgabe der Zusammensetzung ausgelegt ist.

9. Pharmazeutische Zusammensetzung nach Anspruch 5, wobei es sich bei dem pharmazeutisch unbedenklichen Träger um eine Dosiereinheit für die parenterale Verabreichung der colloidalen wässrigen Suspension handelt.

10. Verfahren zur Herstellung einer stabilisierten colloidalen radikalreduzierten wässrigen Suspension aus einem Komplex von Hyaluronsäure (HS) und einem Proteinarzneistoff, **dadurch gekennzeichnet, dass** man die folgenden Schritte unter inerten Bedingungen durchführt:
(a) Lyophilisieren einer wässrigen Lösung einer HS mit einem mittleren Molekulargewicht (mittleren MG) im Bereich von 80 bis 400 kDa und Lyophilisierenlassen während eines Zeitraums, der ausreicht, um die HS in amorpher Form zu erhalten,
(b) Lyophilisieren einer wässrigen Lösung des Proteinarzneistoffs und Lyophilisierenlassen über einen Zeitraum, der ausreicht, um den Proteinarzneistoff in amorpher Form zu erhalten,
(c) Vermischen der lyophilisierten amorphen HS mit einer Mischung aus Elektrolyten und Wasser, wodurch an der HS der erhaltenen homogenen Mischung elektrische Doppelschichten erhalten werden,
(d) Versetzen mit dem lyophilisierten amorphen Proteinarzneistoff bei einem niedrigen pH über einen Zeitraum, der kurz genug ist, um das Protein nicht zu denaturieren, wobei an dem amorphen Proteinarzneistoff elektrische Doppelschichten erhalten werden,
(e) Reduzieren des Wasser- und Ionengehalts auf ein Minimum, wodurch hydrophobe Oberflächen voneinander unter Bildung eines Komplexes aus der amorphen HS und dem amorphen Proteinarzneistoff angezogen werden und wodurch sich der pH des Komplexes erhöht, und
(f) Versetzen mit einem Überschuss an HS mit einem mittleren MG im Bereich von 6-15 kDa in einer Minimalmenge Wasser, um zu der stabilisierten colloidalen Suspension, enthaltend den Komplex aus der amorphen HS und dem amorphen Proteinarzneistoff, zu gelangen.

11. Verfahren nach Anspruch 10, wobei die Lyophilisierung im Schritt (a) bei einer Temperatur von -40°C oder darunter durchgeführt wird.

12. Verfahren nach Anspruch 10, wobei die Lyophilisierung in Schritt (b) bei einer Temperatur von -30°C oder darunter durchgeführt wird.

13. Verfahren nach Anspruch 10, wobei der niedrige pH in Schritt (d) im Bereich von 1-2 liegt und der pH in Schritt (f) auf einen Wert im Bereich von 6-8 erhöht wird.

14. Verfahren nach Anspruch 10, wobei das mittlere MG der HS in Schritt (a) 150 kDa beträgt und das mittlere MG der HS in Schritt (f) 8 kDa beträgt.

15. Verfahren nach Anspruch 10, wobei die Lyophilisierung in Schritt (a) bei einer Temperatur von -50°C oder darunter erfolgt, die Lyophilisierung in Schritt (b) bei einer Temperatur von -45°C oder darunter erfolgt, der pH in Schritt (d) 1,5 beträgt und der pH in Schritt (f) 6,5 beträgt.

16. Verfahren nach Anspruch 10, wobei das Verfahren zusätzlich die Lyophilisierung der in (f) erhaltenen stabilisierten colloidalen wässrigen Suspension zusammen mit zugesetzter HS mit einem mittleren Molekulargewicht von 1-8 kDa unter Bildung eines freifließenden Pulvers umfasst.

17. Verfahren nach Anspruch 10 oder 16, wobei die erhaltene stabilisierte colloidale wässrige Suspension, enthaltend einen Komplex aus amorpher HS und amorphem Proteinarzneistoff, oder das freifließende Pulver davon, in abgebbaren Einzeldosisformen für die orale Verabreichung abgegeben wird.

18. Verfahren nach einem der Ansprüche 10-13, wobei die erhaltene stabiliserte colloidale wässrige Suspension, enthaltend einen Komplex aus amorpher HS und amorphem Proteinarzneistoff, oder eine sterile Lösung davon, in Einzeldosisformen für die parenterale Verabreichung abgegeben wird.

19. Verfahren nach einem der Ansprüche 10-18, wobei der Proteinarzneistoff aus der Gruppe bestehend aus Insulin, Glucagon-Like Peptide 1 (GLP1), menschlichem Wachstumshormon (hGH), Erythropoietin (EPO) und Superoxiddismutase (SOD) stammt.

## Revendications

1. Suspension aqueuse réduite en radical libre, colloïdale, stabilisée contenant un complexe d'acide hyaluronique (HA) amorphe avec un poids moléculaire moyen (MW moyen) dans la plage de 80 à 400 kDa et un médicament protéique amorphe, et une quantité en excès de HA pour réduire au minimum la teneur en eau.

2. Suspension aqueuse colloïdale, stabilisée selon la revendication 1, dans laquelle le HA de la quantité en excès de HA a un MW moyen dans la plage de 6 à 15 kDa.

3. Suspension aqueuse colloïdale, stabilisée selon la revendication 1 ou 2, où la suspension comprend en outre HA avec un MW moyen dans la plage de 1 à 8 kDa et est sous forme de poudre fluide lyophilisée.

4. Suspension aqueuse colloïdale, stabilisée selon l'une quelconque des revendications 1 à 3, dans laquelle le médicament protéique est choisi dans le groupe constitué de l'insuline, GLP-1 (glucagon-like peptide 1), l'hormone de croissance humaine (hGH), l'érythropoïétine (EPO) et la superoxyde dismutase (SOD) .

5. Composition pharmaceutique comprenant une suspension aqueuse colloïdale stabilisée selon l'une quelconque des revendications 1 à 4, et un véhicule pharmaceutiquement acceptable.

6. Composition pharmaceutique selon la revendication 5, dans laquelle le véhicule pharmaceutiquement acceptable est un dispositif doseur par distribution par voie orale pour administration orale de la suspension aqueuse colloïdale.

7. Composition pharmaceutique selon la revendication 5, dans laquelle le véhicule pharmaceutiquement acceptable est une capsule administrable par voie orale.

8. Composition pharmaceutique selon la revendication 7, dans laquelle la capsule est conçue pour administration entérique de la composition.

9. Composition pharmaceutique selon la revendication 5, dans laquelle le véhicule pharmaceutiquement acceptable est un dispositif doseur pour administration parentérale de la suspension aqueuse colloïdale.

10. Procédé de production d'une suspension aqueuse réduite en radical libre, colloïdale, stabilisée d'un complexe d'acide hyaluronique (HA) et un médicament protéique, comprenant la conduite des étapes suivantes dans des conditions inertes :
(a) lyophilisation d'une solution aqueuse de HA ayant un poids moléculaire moyen (MW moyen) dans la plage de 80 à 400 kDa et maintien de la lyophilisation pendant une durée suffisante, pour obtenir le HA sous forme amorphe,
(b) lyophilisation d'une solution aqueuse du médicament protéique et maintien de la lyophilisation pendant une durée suffisante, pour obtenir le médicament protéique sous forme amorphe,
(c) mélange du HA amorphe, lyophilisé avec un mélange d'électrolytes et d'eau, de telle manière que des doubles couches électriques soient obtenues sur le HA du mélange homogène résultant,
(d) ajout du médicament protéique amorphe lyophilisé à un pH faible pendant une durée qui est suffisamment courte pour ne pas dénaturer la protéine, de telle manière que des doubles couches électriques soient obtenues sur le médicament protéique amorphe,
(e) réduction de la teneur en eau et des ions à un minimum, de telle manière que les surfaces hydrophobes soient attirées l'une par l'autre pour former un complexe du HA amorphe et du médicament protéique amorphe ; et de telle manière que le pH du complexe augmente, et
(f) ajout d'une quantité en excès de HA avec un MW moyen dans la plage de 6 à 15 kDa dans le minimum d'eau pour produire la suspension colloïdale stabilisée contenant le complexe du HA amorphe et du médicament protéique amorphe.

11. Procédé selon la revendication 10, dans lequel la lyophilisation dans l'étape (a) est conduite à une température de -40 °C ou plus basse.

12. Procédé selon la revendication 10, dans lequel la lyophilisation dans l'étape (b) est conduite à une température de -30 °C ou plus basse.

13. Procédé selon la revendication 10, dans lequel le pH bas dans l'étape (d) est dans la plage de 1 à 2 et dans l'étape (f) le pH est augmenté à une valeur dans la plage de 6 à 8.

14. Procédé selon la revendication 10, dans lequel le MW moyen du HA dans l'étape (a) est de 150 kDa et le MW moyen du HA dans l'étape (f) est de 8 kDa.

15. Procédé selon la revendication 10, dans lequel la lyophilisation dans l'étape (a) est conduite à une température de -50 °C ou plus basse, la lyophilisation dans l'étape (b) est conduite à une température de -45 °C ou plus basse, le pH dans l'étape (d) est de 1,5 et le pH dans l'étape (f) est de 6,5.

16. Procédé selon la revendication 10, où le procédé comprend en outre la lyophilisation de la suspension aqueuse colloïdale stabilisée obtenue dans (f) conjointement avec HA ajouté avec un poids moléculaire moyen de 1 à 8 kDa, pour obtenir une poudre fluide.

17. Procédé selon la revendication 10 ou 16, dans lequel la suspension aqueuse colloïdale stabilisée obtenue contenant un complexe de HA amorphe et de médicament protéique amorphe ou une poudre fluide de celui-ci est distribuée dans des unités de dose dispositif pouvant être distribuées pour administration orale.

18. Procédé selon l'une quelconque des revendications 10 à 13, dans lequel la suspension aqueuse colloïdale stabilisée obtenue contenant un complexe de HA amorphe et de médicament protéique amorphe obtenu ou une solution stérile de celui-ci est distribuée dans des unités de dose pour administration parentérale.

19. Procédé selon l'une quelconque des revendications 10 à 18, dans lequel le médicament protéique est choisi dans le groupe constitué de l'insuline, GLP-1 (glucagon-like peptide 1), l'hormone de croissance humaine (hGH), l'érythropoïétine (EPO) et la superoxyde dismutase (SOD).
